(19)

Europäisches Patentamt
European Patent Office
Office européen des brevets

(11)  **EP 3 816 276 A1**

(12)  # EUROPEAN PATENT APPLICATION

(43) Date of publication:
05.05.2021 Bulletin 2021/18

(21) Application number: **19206392.3**

(22) Date of filing: **31.10.2019**

(51) Int Cl.:
*C12N 5/071* (2010.01)         *A61L 27/36* (2006.01)
*A61K 35/39* (2015.01)        *A61P 3/10* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Unicyte Islet AG**
**6370 Oberdorf (CH)**

(72) Inventors:
• **NAVARRO TABLEROS, Victor Manuel**
**I-10152 TORINO (IT)**

• **BRIZZI, Maria Felice**
**I-10133 TORINO (IT)**
• **GOMEZ, Yonatan**
**I-10126 TORINO (IT)**
• **CAMUSSI, Giovanni**
**I-10132 TORINO (IT)**

(74) Representative: **Rimini, Rebecca et al**
**Jacobacci & Partners S.p.A.**
**Corso Emilia, 8**
**10152 Torino (IT)**

(54)  **VIABLE PANCREATIC ISLET-LIKE CELL STRUCTURES AND A METHOD OF PREPARING THEREOF**

(57)  The invention relates to a method of preparing pancreatic islet-like cell structures characterized by a unique combination of high viability and morphological and functional features which make them particularly suitable for use in both clinical and drug screening application, as well as to the pancreatic islet-like cell structures obtained therefrom.

EP 3 816 276 A1

**Description**

The present invention relates to the field of stem cell differentiation. In particular, the present invention relates to a method for differentiating a stem cell into a pancreatic islet-like cell structure and to the pancreatic islet-like cell structure obtained therefrom.

Type 1 diabetes (T1DM) is a disease with an important socio-economic impact, caused by autoimmune destruction of pancreatic insulin producing cells ($\beta$-cells) and associated with development of debilitating microvascular and macrovascular complications. Treatment with exogenous insulin to achieve a glycemic control does not completely prevent long term complications. Both pancreas and islets transplantation restore islets function and potentially reduce long-term diabetic complications, but are limited by both donor shortage and need for immunosuppression. Beta-cell replacement is an attractive therapy to achieve normoglycemia in patients with diabetes mellitus and several studies focused on generation of human $\beta$-cells. Pluripotent stem cells for their unlimited capacity of self-renewal may represent a scalable source of insulin producing cells. Beta-like cells have been differentiated from human induced pluripotent stem cells (hiPCS) and human embryonic stem cells (hESC). Nevertheless, most current approaches are complex and require virus-induced transfection or methods of genetic engineering. The challenge of producing $\beta$-cells from pluripotent stem cells faces several inefficiencies including rate of differentiation, generation of immature insulin producing cells, ethical concern, tumors formation or failure to maintain a sustained correction of hyperglycemia. Recently, many efforts have been focused on identifying alternative and abundant cell sources of functional $\beta$-cells. In nature, pancreatic islets contain different endocrine cells: alpha, beta, gamma, epsilon and PP cells that secrete glucagon, insulin, somatostatin, ghrelin and pancreatic polypeptide, respectively. The characteristic conformation of pancreatic islets is important for a fine regulation of the islet function. In particular, it has been demonstrated that paracrine cell interactions among the insular cells help to modulate the hormone secretion and the tuning of normal $\beta$-cell function. Several multistep protocols have been described to differentiate human embryonic or inducible pluripotent stem cells into pancreatic progenitors able to undergo *in vivo* maturation with generation of glucose sensitive insulin secreting cells able to reverse diabetes in mice. The generation of *in vitro* mature endocrine cells would advantage reversal of diabetes. However, progenitors generated *in vitro* are immature and the protocols mainly produce poly-hormonal cells concomitantly expressing insulin, glucagon and somatostatin. Rezania, A., et al., Maturation of human embryonic stem cell-derived pancreatic progenitors into functional islets capable of treating pre-existing diabetes in mice. (2012) Diabetes, 61(8):2016-2029 have recently described a seven-stage protocol that generate glucose responsive insulin secreting cells from human embryonic stem cells. These cells, even if not fully mature, were able to rapidly reverse diabetes.

Insulin producing cells may also be generated from pancreatic progenitors. Despite an immature phenotype with poly-hormonal expression, the cells are able to revert or prevent diabetes in mice. Insulin producing cells have been obtained also by trans-differentiation of adult stem cells, for instance islet-derived mesenchymal stem cells cultured in absence of serum and with an appropriate mix of factors, as well as bone marrow mesenchymal stem cells in conditions mimicking diabetic niche. Other approaches focused on reprogramming pancreatic cell types such as ductal, acinar or alpha cells by inducing epigenetic changes.

Both liver and pancreas share common embryonic origins and the developing liver has been shown to exhibit transcriptional features similar to the adult pancreas. Insulin producing cells were also generated by reprogramming hepatocytes inducing overexpression of PDX1 plus exendin-4 to force proliferation and differentiation. However, a limitation in using human liver hepatocytes is their low proliferative potential *in vitro*. Herrera, M. B. et al. Isolation and characterization of a stem cell population from adult human liver. (2006) Stem Cells. 24, 2840-2850 describes the isolation from the adult human liver by a culture strategy leading death of mature hepatocyte of a cell population with high proliferative capacity defined as adult human liver stem-like cells (HLSC). HLSC are clonogenic and express markers of both mesenchymal stem cells and immature hepatocytes. Moreover, HLSC express several stem cells and embryonic markers, such as nanog, Oct4, SOX2 and SSEA4 and are able to undergo multiple differentiations including islet-like phenotype. However, the differentiation protocol was limited by a very low efficiency and the insulin secretion under glucose control was not demonstrated.

WO2015091493 discloses *in vitro* generation of insulin-producing 3D spheroid structures from an isolated adult stem cell by a protocol based on charge-dependent aggregation promoted by a poly-cationic substance added to the cell culture medium. In particular, WO2015091493 teaches that, following cultivation of isolated adult stem cells in a first differentiation cell culture medium in the presence of a poly-cationic substance, to obtain partially mature pancreatic islet-like cell structures a second cultivation step is required, which is carried out by replacing the first cell culture medium with a second differentiation cell culture medium not comprising a poly-cationic substance.

The present inventors have provided a new method of preparing a spheroid pancreatic islet-like cell structure, which is defined in the appended claims. The content of the claims forms an integral part of the description.

The method according to the present invention results from the finding that culturing isolated adult stem cells in a single culturing passage in the presence of a poly-cationic substance, without changing the cell culture medium, enables to obtain pancreatic islet-like structures which are almost completely functionally mature as well as their increase both in cell number and in structure number. Compared to the prior art, not only the method according to the present invention is simpler, faster and more efficient, but it also results in improved viability of the islets.

Thus, in a first aspect, the invention provides a method of preparing a population of artificially grown spheroid pancreatic islet-like cell structures, comprising culturing an adult stem cell under aerobic condition in a differentiation liquid cell culture medium comprising a poly-cationic substance for a period of time of 5 to 21 days, and then blocking culturing, wherein said culturing is performed in a single culturing passage without changing the differentiation liquid cell culture medium.

Other features and advantages of the method according to the invention are defined in the appended claims which form an integral part of the description.

As described in WO2015091493, isolated adult stem cells aggregate and differentiate into spheroid pancreatic islet-like cell structures when cultured in a liquid cell culture medium in the presence of a poly-cationic substance.

Any poly-cationic substance which is capable of promoting adult stem cell aggregation and differentiation into pancreatic islet cells at a given concentration and which, at that concentration is non-cytotoxic to cells, may be used in the method of the present invention. Illustrative, non-limiting examples of poly-cationic substances suitable for use in the method of the invention are protamine, poly-lysine and cationic dextrans.

Protamine, preferably in the form of a salt, such as for example protamine sulfate or protamine hydrochloride, is the preferred positive polycation because it is suitable for clinical applications, and is added to the medium at a concentration which preferably ranges between 5 and 50 $\mu$g/ml., more preferably between 10 and 30 $\mu$g/ml, even more preferably between 15 and 25 $\mu$g/ml. The most preferred form of protamine is protamine hydrochloride.

The adult stem cell used in the method of the invention is preferably an adult mammalian stem cell. Therefore, any liquid cell culture medium capable of sustaining the growth of mammalian cells is suitable for use in the method of the invention. In one embodiment, the liquid cell culture medium is a serum-enriched cell culture medium. Serum-enriched DMEM or RPMI are mentioned as non-limiting examples. Serum concentration in the cell culture medium is preferably comprised within the range of from 5 to 20%. Preferably, the liquid cell culture medium also comprises one or more carbon sources, such as for example glucose and glutamine. Preferred glucose concentrations are within the range of 6-25 mM. Preferred glutamine concentrations are within the range of 0.5-3 mM.

In another embodiment, the liquid cell culture medium is serum free and serum is replaced by a substitute such as *e.g.* a protein supplement that provides the beneficial growth-promoting activities of albumin and $\alpha$ and $\beta$-globulins. The selection of the most suitable serum substitutes to be used in the method of the invention falls within the knowledge of the person skilled in the art.

Within the context of the present description, the expression "adult stem cell" is intended to mean a stem cell that is isolated from an adult tissue, in contrast with an "embryonic stem cell" which is isolated from the inner cell mass of a blastocyst. Adult stem cells are also known as "somatic stem cells".

According to the method of the invention, the cultivation of spheroid pancreatic islet-like cell clusters is blocked following a cultivation period of 5 to 21 days, preferably a period of time of 5 to 13 days, more preferably a period of time of 5 to 11 days, even more preferably a period of time of 5 to 9 days. Blocking of cultivation may be carried out for example by harvesting the islet-like structures, for example by collecting the supernatant medium containing the islets in suspension and recovering islet-like structures adhering to the culture substrate. It is understood that other methods of blocking islets cultures *in vitro* may be used within the scope of the invention, as are known in the art.

As mentioned above, a key element of the method of the invention is that cultivation of pancreatic islet-like cell structures is carried out over the entire culturing period without changing the differentiation liquid cell culture medium. In this connection it should be understood that the term "changing the differentiation liquid cell culture medium" is intended to encompass replacing the differentiation liquid cell culture medium with a fresh batch of the same liquid medium as well as replacing the differentiation liquid cell culture medium with a different liquid medium. Without wishing to be bound by any theory, the inventors believe that the observed increase in islet number and viability obtained with the method of the invention may be due to the absence of mechanical disturbance of the forming structures during their differentiation as well as to the accumulation of paracrine factors secreted by the cells in the culture medium.

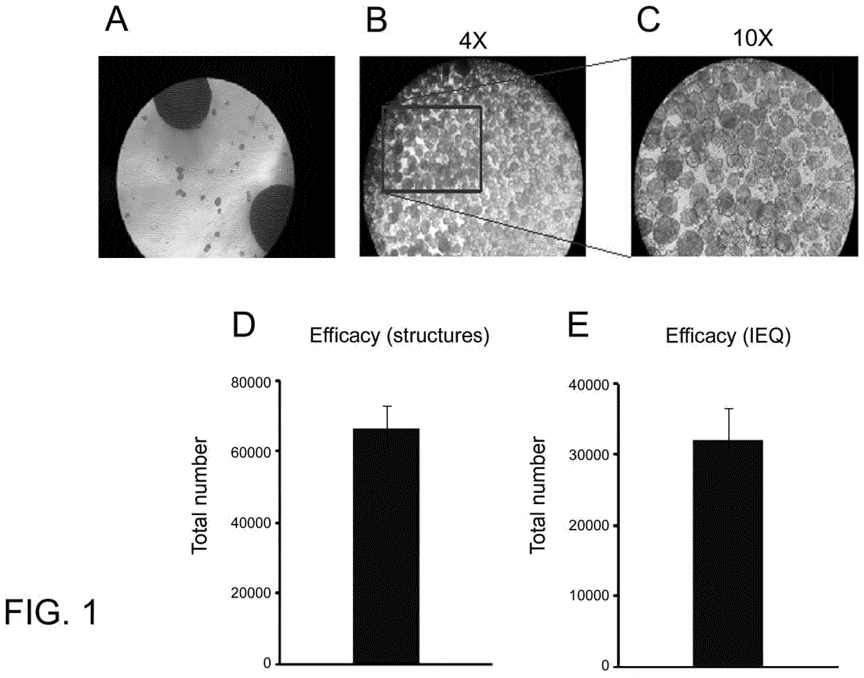

In the method of the invention, the cultivation of pancreatic islet-like cell structures is performed under aerobic condition. As is known in the art, gas exchange, particularly the supply of oxygen and maintenance of constant oxygen levels, are fundamental aspects in cell culture systems in order to maintain culture viability. Indeed, in *in vitro* cultures the oxygen conditions of the microenvironment at the level of cell growth, the so-called pericellular conditions, may substantially influence cellular metabolism and signaling. Titus K. et al, "Closed system cell culture protocol using HYPERStack vessels with gas permeable material technology", J Vis Exp. 2010; (45): 2499 describes in Figure 1 the depletion of pericellular oxygen concentration in culture medium over 3 days in a standard cell culture vessel. For the purpose of the invention, the pericellular concentration of oxygen is preferably of at least 5 mg/l.

Therefore, in a preferred embodiment of the invention, the adult stem cell is cultured on a solid support and the concentration of oxygen in the differentiation liquid cell culture medium at the surface of the solid support is of at least 5 mg/l.

The term "surface" as used herein means the cell growth surface of the solid support.

The cell solid support may be chosen to have any suitable shape and be made from any suitable material capable of sustaining the growth of mammalian cells. Suitable examples may include solid supports comprising, for example, glass, plastic, nitrocellulose or agarose. In one embodiment, the solid support may take the form of a glass or plastic plate or slide or membrane. In other embodiments, the solid support may be a glass or plastic multi-well plate such as, for example a micro-titre plate. In one embodiment the solid support may take the form of a cell culture flask or roller flask. The selection of the most suitable solid support to be used in the method of the invention falls within the knowledge and skills of the person skilled in the art.

In one embodiment of the invention, the concentration of oxygen in the differentiation liquid cell culture medium during the culturing period is comprised within 100% to 80% of the initial oxygen concentration, preferably within 100% to 90%, more preferably within 100% to 95%.

As outlined above, a precise control of oxygen delivery to cells in in vitro culture is fundamental to maintain appropriate pericellular oxygen conditions. Accordingly, in a preferred embodiment of the method of the invention, the solid support employed for cell culturing is gas permeable. This embodiment is based on the fact that a permeable support allows direct diffusion of oxygen from the bottom of the support, bypassing the diffusion barrier created by the culture medium above the cells.

Preferably, the cell culture support is a gas permeable membrane. Gas permeable membrane materials may be made from any suitable gas permeable material so long as it provides free passage of gases such as oxygen and carbon dioxide through the solid support. Non-limiting examples of suitable gas permeable material include polystyrene, polyethylene, polycarbonate, polyolefin, ethylene vinyl acetate, polypropylene, polysulfone, polytetrafluoroethylene (PTFE) or compatible fluoropolymer, a silicone rubber or copolymer, poly(styrene-butadiene-styrene) or combinations of these materials.

In a preferred embodiment of the invention, the thickness of the gas permeable membrane employed as cell culture support is comprised within the range of from 25 $\mu$m to 250 $\mu$m, more preferably within the range of from 25 $\mu$m to 125 $\mu$m.

The permeability of gases in a polymer material may vary depending on the material properties and permeants. Preferably, in the method according to the invention the permeability of the gas permeable membrane is of at least 1000 (g $25\mu/m^2/24h$) as expressed in grams per square meter per day.

In another embodiment of the method of the invention, the solid support is made of a polymeric material modified in order to enhance significantly cell attachment thereto compared to the unmodified polymeric material.

The term "significantly" as used herein means that there is a statistically significant difference in cell adhesion ability between the modified polymeric material of the solid support and the unmodified polymeric material as evaluated by setting the statistical significance level at 0.05 (p-value < 0.05). Within this context, statistical analysis was conducted by using any of the known suitable statistical methods known in the prior art such as ANOVA and t-test.

Exemplary polymeric materials suitable for use in the present invention include but are not limited to polyacrylates, polymethylacrylates, polycarbonates, polystyrenes, polysulphones, polyhydroxy acids, polyanhydrides, polyorthoesters, polyphosphazenes, polyphosphates, polyesters, nylons or mixtures thereof.

By way of example, the polymeric material of the solid support may be polystyrene modified so as to increase the oxygen-containing functional groups of the polystyrene backbone, thereby providing superior wettability, hydrophilicity and ECM proteins adhesion to the surface.

In a more preferred embodiment of the invention, the adult stem cell is cultured in a culture vessel comprising a plurality of layered cell culture solid supports.

In this embodiment, the multilayered arrangement of cell culture solid supports, preferably gas permeable membranes, assembled into a unitary vessel provides a significantly increased surface area for cell growth thereby allowing to significantly increase the yield of pancreatic islet-like cell clusters obtainable with the method of the invention.

The cell culture vessel may be for example a flask, for instance a polystyrene flask, in which a plurality of gas permeable membranes is disposed in a stacked arrangement. Oxygen can be introduced to the multilayered vessel by means of one or more vented caps or directly through the thin surfaces of integrated air spaces. The cell culture supports utilized in the embodiments of the present invention may be capable of gas exchange, achieving uniform gaseous distribution throughout the cell culture vessel. Exemplary cell culture multilayered vessels are described in US 8,178,345 and US 7,745,209, whose content is incorporated herein by reference in its entirety.

According to another preferred embodiment of the invention, the adult stem cell which is differentiated into pancreatic islet-like cell structures is an adult human liver stem cell (HLSC). A preferred human liver stem cell is the human non-oval liver stem cell (HLSC) expressing both mesenchymal and embryonic stem cell markers disclosed in WO 2006/126219. This cell line is in particular characterized in that it is a non-oval human liver pluripotent progenitor cell line which is isolated from adult tissue, which expresses hepatic cell markers and which has multipotent differentiation abilities and regenerative properties. More in particular, this cell line is capable of differentiating into mature liver cells, insulin producing cells, osteogenic cells and epithelial cells. According to a preferred embodiment, it expresses one or more markers selected from the group comprising albumin, a- fetoprotein, CK18, CD44, CD29, CD73, CD146, CD105, CD90 and any combination thereof, and it does not express markers selected from the group comprising CD 133, CD117, CK19, CD34, cytochrome P450.

The human non-oval liver pluripotent progenitor/stem cells disclosed in WO 2006/126236 were shown to undergo differentiation into a variety of tissue cell types (namely, mature liver cells, epithelial cells, insulin-producing cells and osteogenic cells) and to exert organ regenerating effects. Such cells are derived from a non-oval human liver pluripotent progenitor cell line which expresses hepatic cell markers. Such cells are isolated by a method comprising the steps of:

(i) culturing adult liver-derived human mature hepatocytes in a cell culture medium until death of mature hepatocytes and selection of a population of surviving cells having epithelioid morphology;

(ii) expanding the population of surviving cells having epithelioid morphology by culturing in a serum-containing, glucose-containing culture medium supplemented with hEGF (human epithelial growth factor) and bFGF (basic fibroblast growth factor) and comprising the usual inorganic salts, amino acids and vitamins necessary for the growth of mammalian cells and in particular wherein the mature hepatocytes are frozen in a serum-containing culture medium in the presence of a cryoprotecting agent and then thawed prior to culturing according to step (i).

The characterization of the human non-oval liver stem/progenitor cells disclosed in WO 2006/126236 and the method of preparing thereof are herein fully incorporated by reference.

The use of HLSCs in the present invention is preferred for a number of reasons: 1) they are relatively easy to isolate and expand, 2) they are characterized by a degree of proliferation that allows to provide a suitable number of cells for therapeutic use, 3) they can be used autologously and 4) they avoid ethical concerns. Furthermore, the liver and the pancreas share common embryonic origins and the developing liver has been shown to exhibit transcriptional features similar to the adult pancreas (Bose B et al. Cell Biol Int. 2012; 36(11): 1013-20). However, since it is envisaged that poly-cationic substances shall be effective in promoting aggregation and differentiation of other adult stem cells than HLSCs, the scope of the invention is not limited to the use of HLSCs only, but it includes the use of any adult stem cells type.

As mentioned above, the pancreatic islet-like cell structures obtainable by the method of the present invention are characterized by a unique combination of high viability and *in vitro* functional properties. Accordingly, the scope of the invention also includes a composition comprising a population of artificially grown spheroid pancreatic islet-like cell structures as defined in the appended claims.

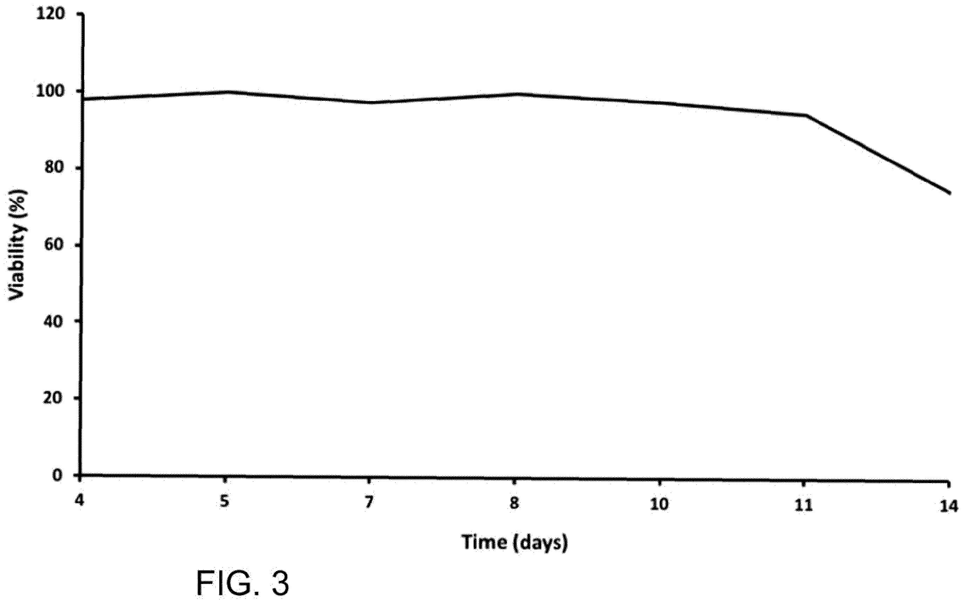

As further illustrated in the experimental section which follows, viability assessment conducted by double staining with fluorescein diacetate (FDA) and propidium iodide (PI) revealed that islet-like structures remain more than 93% viable following over several days of *in vitro* culturing according to the invention (Figure 3).

Therefore, the population of spheroid pancreatic islet-like structures according to the present invention are advantageously characterized by a viability above 93% as measured by the fluorescein diacetate (FDA) and propidium iodide (PI) assay. In a preferred embodiment of the invention, the population of spheroid pancreatic islet-like structures shows a viability above 95 %, more preferably above 97%, even more preferably above 98%.

The spheroid pancreatic islet-like structures according to the present invention are further characterized by enhanced glucose-responsiveness as measured by the ability of secreting C-peptide in response to high glucose (HG) stimulation in an amount which is at least two-fold the amount secreted in response to low glucose (LG) stimulation in both static or dynamic conditions.

In a preferred embodiment of the invention, the amount of C-peptide secreted in response to high glucose (HG) stimulation is of at least 50 pg/ml/400 IEQ, preferably at least 80 pg/ml/400 IEQ, more preferably at least 95 pg/ml/400 IEQ, even more preferably at least 100 pg/ml/400 IEQ.

Within the context of the present description, the term "high glucose" is intended to refer to a stimulus of 28 mM glucose, whereas the term "low glucose" is intended to refer to a stimulus of up to 2.8 mM glucose, excluding 0 value, for example from 0.1 mM up to 2.8 mM glucose.

In one preferred embodiment of the invention, at least 50% of the spheroid pancreatic islet-like cell structures in the population have a diameter of less than 150 $\mu$m, preferably equal to or less than 100 $\mu$m, more preferably of from 51 to 100 $\mu$m.

According to another preferred embodiment of the invention, the mean diameter in the population of spheroid pancreatic islet-like cell structures is less than 100.

According to the present invention, the spheroid pancreatic islet-like structures express at the protein level the pancreatic hormones which are usually produced by human pancreatic islets (*i.e.* insulin, glucagon, pancreatic polypeptide, somatostatin and ghrelin)..

In one preferred embodiment of the invention, the pancreatic islet-like structures according to the invention express one or more additional markers of pancreatic $\beta$-cell development and/or function such as, for example, the $\beta$-

cell transcription factors Nkx6.1, Nkx6.3, MafA and MafB, the endocrine specific markers chromogranin A (CgA) and Ngn3, and the exocrine pancreatic markers PDX1 and synaptophysin, and any combination thereof.

Within the context of the present description, IEQ/100 ILS is the Islets Equivalent of an average diameter of 150 $\mu m$ (IEQ) normalized to 100 islets (ILS). According to conventional protocols, the IEQ of islets isolated from the pancreas and used for transplantation is determined as follows. Suspended islets isolated from the pancreas are evaluated both in number and size (diameter) in order to determine the total islets equivalent (i.e. total islets volume). Islets diameter assessment takes into account 50 $\mu m$ diameter range increments, from 51 $\mu m$ to >350 $\mu m$, without taking into account diameters < 50 $\mu m$, that do not provide a significant contribution to the total volume. A relative conversion factor is conventionally used to convert the total islets number to Islets Equivalent of an average diameter of 150 $\mu m$ (IEQ).

According to the above-illustrated morphological, functional and viability properties, the pancreatic islet-like structures according to the invention are particularly suitable for use both in clinical application, such as in a method treatment of diabetes by pancreatic islet transplantation, and in *in vitro* applications, such as screening methods for identifying substances capable of promoting the expression of one or more pancreatic hormones by pancreatic islet cells or for identifying substances capable of exerting a cytotoxic effect on pancreatic islet cells.

The following experimental section is provided purely by way of illustration and is not intended to limit the scope of the invention as defined in the appended claims. In the following experimental section reference is made to the appended drawings, wherein:

Figure 1: *in vitro* differentiation of HLSC into islet-like structures (HLSC-ILS). Representative micrographs showing HLSC-ILS at day 7 of in vitro culturing, before (A) and after (B, C) collection from the Hyperflask. (B) 4x micrograph; (C) 10x micrograph. (D, E) graphs showing the results of efficacy analysis measured as total number of HLSC-ILS (D) or IEQ (E). Data are shown as Media $\pm$ SD of 9 independent experiments.

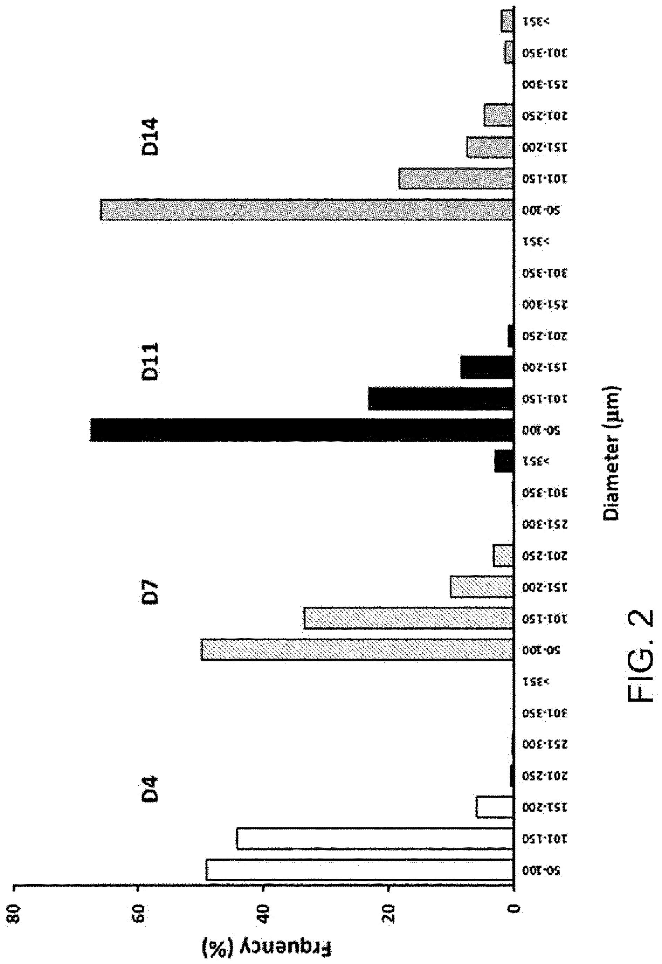

Figure 2: graph showing the size distribution of islet-like structures derived from HLSCs during in vitro culturing (4, 7, 11 and 14 days). Data are expressed as relative frequency.

Figure 3: graph showing HLSC-ILS viability over *in vitro* culturing, from day 4 to day 14, as measured by FDA/PI staining. Percentage of viable islets was calculated as percentage alive (FDA)/percentage dead (PI).

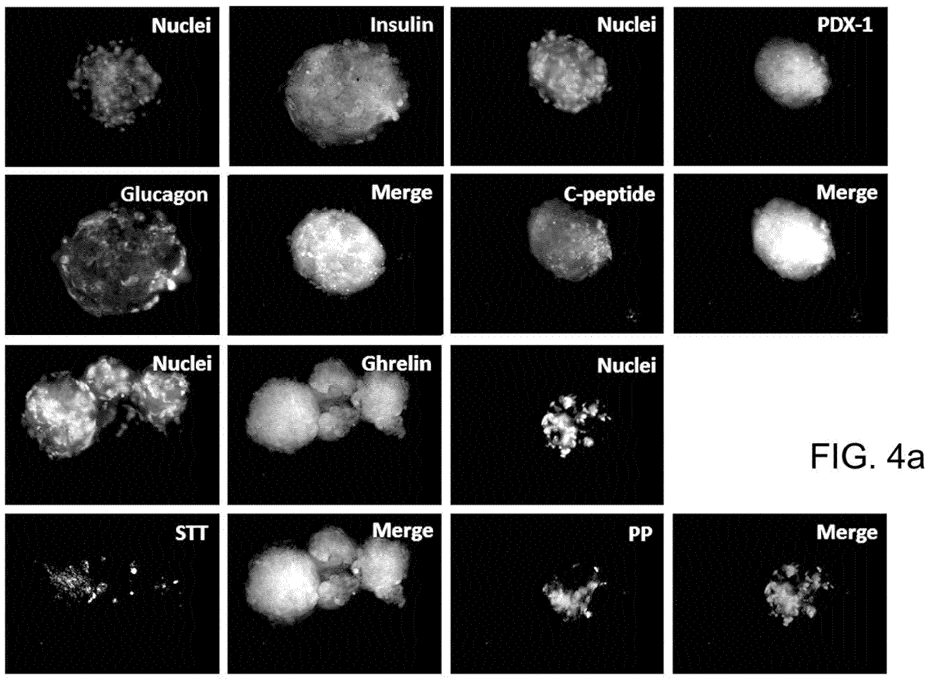

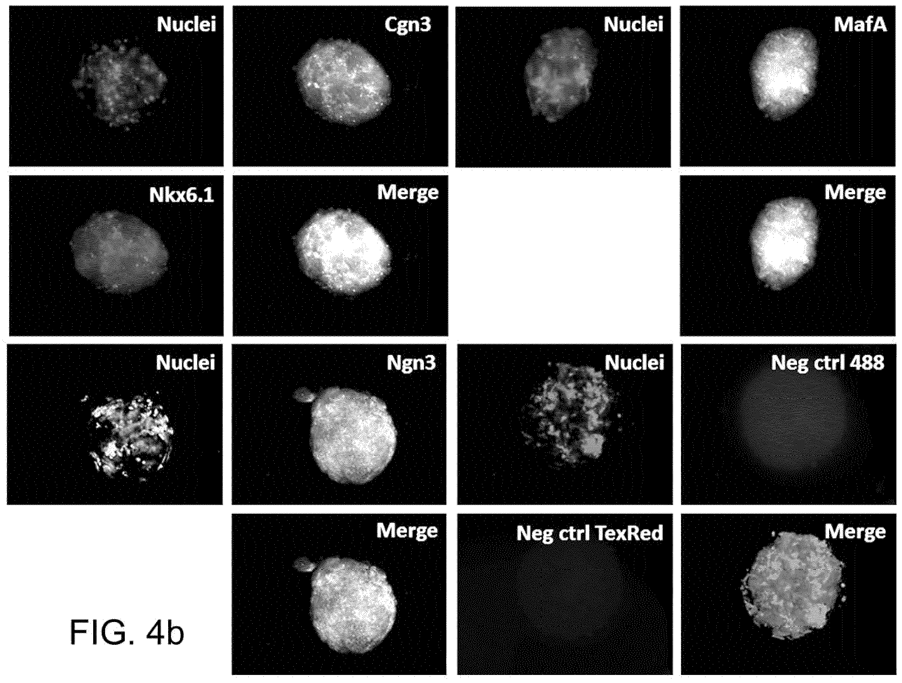

Figure 4: representative pictures showing islet-like structures characterization by immunofluorescence. Islets-like structures become positively stained for (A) insulin, PDX-1, glucagon, C-peptide, ghrelin, somatostatin (STT), pancreatic polypeptide (PP), and (B) Cgn3, MafA, Nkx6.1, Ngn3. Negative controls: Neg ctrl 488 and Neg ctrl TexRed

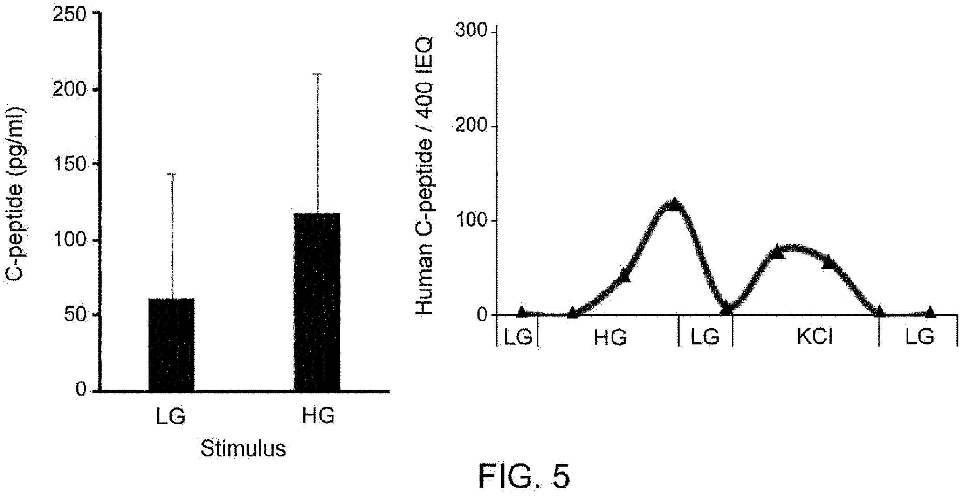

Figure 5: human C-peptide (hC-peptide) *in vitro* secretion by HLSC-ILS in static and dynamic conditions.in response to low glucose (LG) and high glucose (HG) stimulation. (A) Graph showing that at day 7 HLSC-ILS (400 IEQ) secreted hC-peptide in basal conditions of glucose (LG) with a further increase in response to high glucose stimulus (HG). n = 3 independent experiments. (B) Graph showing dynamic secretion of hC-peptide by HLSC-ILS at day 14 of in vitro culturing following a single pulse stimulation of high glucose.

Examples

1. Isolation, characterization and culture of Human Liver Stem Cells (HLSC)

HLSCs were isolated from human cryopreserved normal hepatocytes obtained from Lonza Bioscience (Basel, Switzerland) and cultured and characterized as previously described (Herrera, M. B. et al., Isolation and characterization of a stem cell population from adult human liver. (2006) Stem Cells. 24: 2840-2850.). Cadaveric human islets used as control were obtained from Tebu-bio (Magenta, Italy).

2. *In vitro* differentiation of HLSC into Islet-Like Structures (HLSC-ILS)

Cells thawing and expansion

The cryovials containing the HLSC (P7-9) were thawed in water bath at 37°C. Cell suspension was transferred to a 15 ml tube containing 10 ml of RPMI 1650 supplemented with L-glutamine (5 mM), 50 IU/ml penicillin / 50 $\mu g$/ml streptomycin and 10 % FBS (RPMI 10 % FBS). Cells were centrifuged at 1200 rpm for 5 minutes, the supernatant was discarded, total cells were counted and plated $10 \times 10^6$ cells/ HYPERflask (Corning Ref: 10034) in 560 ml of GMP-like basal HLSC medium having the following composition:

- 500 ml $\alpha$-minimum essential medium (a-MEM; LONZA. Ref. BE12-169F)
- 50 ml of decomplemented FBS
- 250 $\mu$l of FGF2-EGF each (250 mg/ml-250 mg/ml)
- L-glutamine (5 mM)
- Penicillin (50 IU/ml), streptomycin (50 $\mu$g/ml).

Plated cells were incubated in a humidified 5% $CO_2$ incubator at 37°C and maintained in culture until ≈ 70-80% of confluence.

Detachment of cells

When the cells were approximately ≈ 80% confluent, the supernatant was discarded and the cells were washed twice with PBS IX to eliminate medium remnants. A total of 560 ml of Triple (Gibco. REF. 12563-029) diluted in PBS IX (1:5) was added and cells were incubated at 37°C for 5 minutes in incubator. The flasks were shaken softly and manually until all cells were detached and the cell suspension was transferred in a sterile bottle. The HYPERflask was washed with 200 ml of RPMI-1640.

The cell suspension was divided in 50 ml tubes and centrifuged at 1200 rpm for 5 minutes high acceleration/high deceleration. After discarding the supernatant, the cell pellets were resuspended with the remnant liquid and transferred in a single 10 ml tube all together. The total volume and total number of cells were quantified as follows: 90 $\mu$l of Methylene Blue (1:10) + 10 $\mu$l of cell suspension.

HLSC-ILS differentiation

To promote cell differentiation, the inventors employed RFG medium (560 ml for each HYPERflask): RPMI 10 % FBS supplemented with glucose 11.6 mM (5.8 ml of 1M glucose solution). About 70-80 x $10^6$ HLSC were resuspended in 280 ml of RFG medium. The cell solution was transferred into the HYPERflask avoiding bubble formation and the closed HYPERFLASK was placed horizontally in order to distribute the volume homogenously in all ten levels. Then, the hyperflask was placed vertically and filled up with the remnant RFG solution (280 ml). 1ml of protamine solution (10 mg/ml) was added to a final concentration of 18 $\mu$g/ml and the HYPERflask was shaken manually, gently, to distribute the Protamine solution. The HYPERflask was incubated in a humidified 5% $CO_2$ incubator at 37°C for 7 days, without changing the medium.

The formation of HLSC-ILS usually starts after the first 12-48 hours, and to obtain successful aggregation the cells shall not be disturbed during the next 4 days following stimulation. Indeed, any mechanical stress may interfere with size homogeneity and number of generated HLSC-ILS.

HLSC-ILS collection

At day 4-14 post-stimulation, the HLSC-ILS were collected by shaking the HYPERflask manually and gently to detach the islets from the wall surface. The entire volume from the HYPERflask was transferred directly into a sterile bottle. Then, the volume was divided in 50 ml tubes and left for 10 minutes to obtain a stress-free precipitation of the HLSC-ILS. The tubes were centrifuged at 1000 rpm for 5 minutes, the supernatant was aspirated by using a 10 or 25 ml pipette leaving ~ 2 ml of liquid, which was gently resuspended to obtain a homogeneous HLSC-ILS suspension.

3. Transmission electron microscopy

Transmission electron microscopy was performed on Karnovsky's-fixed, osmium tetraoxide-postfixed tissues and embedded in epoxy resin, according to standard procedures. Ultra-thin sections were stained with uranyl acetate and lead citrate and were examined with a Jeol JEM 1010 electron microscope (Jeol). By transmission electron microscopy, endocrine granules typical of human islets were observed in the 3D spheroidal structures during *in vitro* culturing. Three main types of electron-dense granules were observed: granules containing crystalloids, granules with a diffuse gray pale core, and granules with a dense core.

4. Efficacy assessment

For each HLSC-ILS preparation, the present inventors determined both the efficacy, i.e. the number of HLSC-ILS/batch generated according to the method of the invention (Figure ID) and the islet equivalents (IEQ)/HLSC-ILS (Figure IE).

To assess the efficacy of preparation, at the end of culture time HLSC-ILS were mechanically detached (handily shake), and the suspension thus obtained was divided in 50 ml tubes and centrifuged at 1000 rpm for 5 minutes. Then, the supernatant was discarded and HLSC-ILS were collected in a single tube. The total volume of HLSC-ILS suspension ($\mu$l) was measured along with the number of HLSC-ILS in a sample of 50-100 $\mu$l, and the efficacy was calculated as follows:

$$\text{Efficacy} = \text{Total number of HLSC-ILS in the sample} \times \text{Total volume of HLSC-ILS suspension /sample volume.}$$

To assess the islet equivalents (IEQ)/HLSC-ILS, the single maximum diameter was determined in each single HLSC-ILS present in a sample ($\sim$ 100 HLSC-ILS/preparation). Then, the HLSC-ILS were distributed in groups according to size ranges. The number of Islet equivalents (IEQ) in the sample were then calculated by multiplying the total number of HLSC-ILS in each subpopulation by an established equivalent factor. Additionally, the size distribution (Figure 2) was obtained by calculating the percentage of each population. An example of the above illustrated calculations is shown in Table 1 below:

| Size (um) | IEQ conversion factor (IEDCF) | **Number of HLSC-ILS** (Efficacy) | **IEQ** (Efficacy) | % (size distribution) |
|---|---|---|---|---|
| 50-100 | 0,167 | **62** | **10,4** | **56,9** |
| 101-150 | 0,648 | **41** | **26,6** | **37,6** |
| 151-200 | 1,685 | **5** | **8,4** | **4,6** |
| 201-250 | 3,5 | **0** | **0,0** | **0,0** |
| 251-300 | 6,315 | **1** | **6,3** | **0,9** |
| 301-350 | 10,352 | **0** | **0,0** | **0,0** |
| <351 | 15,833 | **0** | **0,0** | **0,0** |
| | Total | **109,0** | **51,7** | **100,0** |
| | TOTAL HLSC-ILS | | **56000** | |
| | TOTAL IEQ/batch | | **26541,9** | |

5. Size frequency distribution

Micrographs were obtained using a Cell Observer SD-ApoTome laser scanning systems (Carl Zeiss International, Jena, Germany) using a 10X objective. Almost 500 HLSC-ILS were randomly selected from each of four different experiments and the major diameter was measured for each structure as described in Ricordi, C., et al. (1990). "Islet isolation assessment in man and large animals", Acta Diabetologica Latina, 27: 185-195. Thereafter, the size frequency distribution of the diameters was calculated using the AxioVision software Rel3.4 (Zeiss, Germany). As shown in Figure 2, in *in vitro* cultures at days 4, 7, 11 and 14 more than 50% of HLSC-ILS had a diameter comprised between 50 and 100 $\mu$m.

6. Viability assay

For viability assessment, stock solutions of propidium iodide (PI) (1 mg/ml in PBS 1X) and Fluorescein Diacetate (FDA) (1 mg/ml in acetone) were prepared. Working dilutions were then prepared by diluting IP stock solution 1:20 in PBS 1X, and FDA stock solution 1:100 in PBS 1X, and dilutions were used within 30 minutes.

HLSC-ILS were centrifuged at 1000 rpm for 5 minutes and the islet pellet was seeded in a 24-well plate with 200 $\mu$l of PBS 1X. The islets were subsequently incubated for 30 seconds with 20 $\mu$l diluted FDA and 20 $\mu$l diluted PI solutions, and micrographs taken. Dead cells were stained in RED whereas viable cells were stained in GREEN. HLSC-ILS were then assigned to the following categories:

Cat.0: few/n° green cells (non viable)
Cat.1: 75% red cells (25% viability)
Cat.2: 50% red cells (50% viability)
Cat.3: 25% red cells (75% viability)

Cat.4: few/n° red cells (100% viability).

Total viable islets were then calculated as follows: (0.25 x n°. Cat.1) + (0.5 x n°. Cat.2) + (0.75 x n°. Cat.3) + n°. Cat.4. Total islets number was calculated as follows: n°. Cat.0 + n° . Cat.1 + n° . Cat.2 + n° . Cat.3 + n° . Cat.4. N° corresponds to the number of HLSC-ILS in each category. The percentage of viability was determined by applying the formula: Total viable HLSC-ILS x 100 / Total n° of HLSC-ILS.

As shown in Figure 2, the highest proportion of generated HLSC-ILS have a diameter comprised between 50 $\mu$m and 150 $\mu$m. A small population of islets has a diameter within 151 $\mu$m and 250 $\mu$m and a very low number of structures have a diameter > 250 $\mu$m.

### 7. Immunocytochemistry

The expression of pancreatic markers was assessed by immunofluorescence in HLSC-ILS during culturing. At the end of the experiments HLSC-ILS were collected and seed in positive-charged glass slides and incubated for 60 minutes in incubator to promote their attachment. Then fixed in paraformaldehyde 4% (PAF, overnight at 4°). Briefly, samples were first washed with PBS 1×, twice, incubated for 5 minutes at 4°C with a permeable solution containing 20 mmol/l Hepes, 50 mmol/L NaCL, 300 mmol/L sucrose, 3 mmol/L MagC12, 0,5 % Triton X- 100, pH 7.4. After washing with PBS, the slices were incubated for 1 hour at room temperature with a blocking solution of PBS added with 3% bovine albumin and incubated overnight at 4°C with, washed twice with PBS 1 × for 5 min each one, incubated for 20 min with a blocking solution containing PBS 1 ×, Tween (0.1%), and 0.1% bovine serum albumin (wt/vol) for 30 minutes at room temperature, and then incubated overnight with specific primary antibodies or irrelevant isotype controls. Sections were incubated overnight with the following primary anti-human proteins antibodies: PDX1 (1:500), Ngn3 (1:50), MafB (2 $\mu$g/ml), Nkx6.1 (1:50), Nkx6.3 (2 $\mu$g/ml), chromogranin A (CgA) (2 $\mu$g/ml), C-peptide (2$\mu$g/ml), glucagon (1:50), somatostatin (2 $\mu$g/ml), pancreatic polypeptide (3 $\mu$g/ml) and ghrelin (3 $\mu$g/ml) (1:200) (Abcam, Cambridge, MA); insulin (1:200, Dako, Copenhagen, Denmark); Glut-2 (1:200, Santa Cruz, Santa Cruz, TX). An appropriate isotopic irrelevant antibody (Abcam) was used as control. Following washing with PBS-Tween solution, sections were incubated with an appropriate secondary antibody 1:1000 (Alexa Fluor 488 Donkey anti-goat IgG for PDX1 and PP; Goat anti-Mouse IgG for CgA, C-peptide, ghrelin; Goat anti-Rabbit IgG for MafB, Nkx6.1, Nkx6.3, Glut-2, glucagon, somatostatin, Goat anti-Guinea pig for insulin; Texas Red Goat anti-Mouse IgG for Ngn3 (Invitrogen, Carlsbad, CA) at room temperature for one hour, washed with PBS-tween solution and incubated 10 minutes with DAPI (Dako). After a washing step with PBS, slides were mounted with Fluoromount (Sigma). Specificity of the primary antibodies recognizing human markers was verified by omitting the primary antibodies or by substitution with non-immune isotopic control. Confocal microscopy analysis was performed using a Zeiss LSM 5 Pascal Model Confocal Microscope (Carl Zeiss International).

As shown in Figure 4, immunofluorescence analysis revealed that HLSC-ILS expressed the $\beta$-cell transcription factors Nkx6.1, and the endocrine specific marker Ngn3 at 14 days of differentiation. Furthermore, at the same time-point HLSC-ILS expressed the exocrine pancreatic marker PDX1 and the human islet specific hormones, including insulin/C-peptide, glucagon, somatostatin and ghrelin. All isotype controls were negative.

### 8. *In vitro* glucose-stimulated static assay

To examine static hC-peptide secretion by the pancreatic islet-like structures according to the invention in response to glucose, the present inventors carried out incubations of the islet-like structures under static conditions. Briefly, HLSC-ILS were initially stimulated with a basal concentration of glucose (2.8 mM; LG1) for 1 hour, then the islets were stimulated with high glucose (28 mM; HG) for 2 hours followed by stimulation with high potassium (50 mM; KCl) for 1 hour. Both glucose and KCl stimulation steps were separated by 1-hour incubation with low glucose (LG2). The static assay terminated with a 1 hour-stimulation with 2.8 mM glucose (LG3).

### 9. In vitro dynamic glucose-stimulated secretion

To investigate the dynamics of glucose-stimulated hC-peptide secretory response of the pancreatic islet-like structures according to the invention, the present inventors employed a dynamic perfusion system, more particularly a Microfluidic Dynamic perfusion device (DPD). Briefly, C-peptide secretion by HLSC-ILS was induced at day 7 and 14 of culturing with a single pulse glucose stimulation and potassium or with a constant high glucose stimulation.

In the single pulse protocol, the concentrations of the solutions employed for stimulation were as follows: 2.8 mM glucose (basal glucose, LG), 28 mM glucose (high glucose, HG) and 50 mM potassium chloride (KCl). Flow rate was set to 30 $\mu$l/minute. The following stimulation sequence was applied:

1. 20 minutes with LG (LG1)

2. 15 minutes of transition from LG to HG
3. 30 minutes with HG
4. 15 minutes of transition from HG to LG
5. 10 minutes with LG
6. 15 minutes of transition from LG to KCl
7. 15 minutes with KCl
8. 20 minutes of transition from KCl to LG
9. 10 minutes with LG.

In the constant high glucose stimulation protocol, the concentrations of the solutions employed for stimulation were as follows: 2.8 mM glucose (basal glucose, LG) and 17 mM glucose (high glucose, HG). Flow rate was set to 30 μl/minute. HLSC-ILS were stimulated by applying the following sequence of steps:

1. 20 minutes with LG
2. 65 minutes with HG

10. Quantitative determination of C-peptide

For the quantitative determination of C-peptide in the perfusates, the inventors employed the ALPCO KIT ELISA (Alpco Diagnosis, Windham, NH). Briefly, 25 μl of each standard, control, and sample were added with 50 μl of the proprietary Assay Buffer to the 96-well microplate coated with a monoclonal antibody specific for C-peptide. The microplate was then incubated for 1 hour at room temperature on a microplate shaker at 700-900 rpm. After the first incubation was complete, the wells were washed 6 times with 200 μl of Working Strength Wash Buffer per well and blotted dry. A total of 100 μl of Working Strength Conjugate were then added to each well, and the microplate was incubated a second time on a microplate shaker at 700-900 rpm for 1 hour, washed (6 times with 350 μl of Working Strength Wash Buffer), and blotted dry. TMB Substrate was added to each well (100 μl), and the microplate was incubated a third time for 15 minutes at room temperature on a microplate shaker at 700-900 rpm. Once the third incubation was complete, 100 μl of Stop Solution were added into each well, and the optical density (OD) was measured by a spectrophotometer at 450 nm. The intensity of the color generated was directly proportional to the amount of C-peptide in the sample.

11. Statistical Analysis

The number of experiments is reported in the figure legends. All data were analyzed with GraphPad Prisma software and presented as mean $\pm$ standard deviation (SD). Student's t test was used for the comparison between two groups. When more than two groups were studied, data were analyzed by ANOVA and if significant, the Newman-Keuls, or Dunnet, or Kruskal-Wallis multi-comparison tests were used when appropriate. P values <0.05 were considered statistically significant.

**Claims**

1. A composition comprising a population of artificially grown spheroid pancreatic islet-like cell structures which comprise differentiated adult stem cells expressing at the protein level the pancreatic hormones insulin, glucagon, pancreatic polypeptide, somatostatin and ghrelin, **characterized in that** the differentiated cells are capable of producing an amount of pancreatic C-peptide in response to high glucose (HG) stimulation which is at least two-fold the amount produced in response to low glucose (LG) stimulation in both static or dynamic conditions, and further **characterized in that** the viability of the spheroid pancreatic islet-like cell structures in the population is above 93% as measured by the fluorescein diacetate (FDA) and propidium iodide (PI) assay.

2. The composition according to claim 1, wherein the differentiated cells are capable of producing pancreatic C-peptide in an amount of at least 50 pg/ml/400 IEQ in response to HG stimulation in both static or dynamic condition.

3. The composition according to claim 1 or 2, wherein at least 50% of the spheroid pancreatic islet-like cell structures in the population have a diameter equal to or less than 100 μm, preferably of from 51 to 100 μm.

4. The composition according to any of claims 1 to 3, wherein the mean diameter in the population of spheroid pancreatic islet-like cell structures is less than 100 μm.

5. The composition according to any of claims 1 to 4, wherein said high-glucose stimulation of spheroid pancreatic islet-like cell structures is performed with 28 mM glucose.

6. The composition according to any of claims 1 to 5, wherein the differentiated cells further express at least one marker selected from the group consisting of PDX1, Ngn3, chromogranin A (CgA), Nkx6.1, MafA and any combination thereof.

7. The composition according to any of claims 1 to 6, wherein the adult stem cell is a human liver stem cell (HLSC).

8. The composition according to any of claims 1 to 7, for use in the treatment of diabetes by pancreatic islet transplantation.

9. A method of preparing a population of artificially grown spheroid pancreatic islet-like cell structures, comprising culturing an adult stem cell under aerobic condition in a differentiation liquid cell culture medium comprising a poly-cationic substance for a period of time of 5 to 21 days, and then blocking culturing, wherein said culturing is performed in a single culturing passage without changing the differentiation liquid cell culture medium.

10. The method according to claim 9, wherein culturing the adult stem cell in the differentiation liquid cell culture medium is performed for a period of time of 5 to 13 days, preferably for a period of time of 5 to 11 days.

11. The method according to claim 9 or 10, wherein the adult stem cell is cultured on a solid support and the concentration of oxygen in the differentiation liquid cell culture medium at the surface of the solid support is of at least 5 mg/l.

12. The method according to claim 11, wherein the concentration of oxygen in the differentiation liquid cell culture medium during the culturing period is comprised within 100% to 80% of the initial oxygen concentration.

13. The method according to any of claims 9 to 12, wherein the poly-cationic substance is a soluble salt of protamine or polylysine.

14. The method according to claim 13, wherein the soluble salt of protamine is protamine sulfate or protamine hydrochloride.

15. The method according to claim 14, wherein the protamine sulfate or protamine hydrochloride is at a concentration comprised within 5 to 50 $\mu$g/ml, preferably within 10 and 30 $\mu$g/ml.

16. The method according to any of claims 11 to 15, wherein the solid support is a gas permeable support.

17. The method according to any of claims 11 to 16, wherein the solid support is made of a polymeric material modified so that cell attachment thereto is significantly enhanced compared to the unmodified polymeric material.

18. The method according to any of claims 11 to 17, wherein the adult stem cell is cultured in a culture vessel comprising a plurality of layered cell culture solid supports.

19. The method according to any of claims 9 to 18, wherein the adult stem cell is a human liver stem cell (HLSC).

20. The use of a composition comprising a population of spheroid pancreatic islet-like cell structures according to any of claims 1 to 7, in an *in vitro* screening method for identifying substances capable of promoting the expression of one or more pancreatic hormones by pancreatic islet cells or for identifying substances capable of exerting a cytotoxic effect on pancreatic islet cells.

FIG. 1

FIG. 2

FIG. 3

EP 3 816 276 A1

FIG. 4a

FIG. 4b

FIG. 5

EP 3 816 276 A1

Europäisches
Patentamt

European
Patent Office

Office européen
des brevets

# EUROPEAN SEARCH REPORT

Application Number

EP 19 20 6392

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | WO 2015/091493 A1 (FRESENIUS MEDICAL CARE DE GMBH [DE]) 25 June 2015 (2015-06-25) | 1-8,20 | INV.<br>C12N5/071 |
| Y | * page 3 - page 4; claims 1-3 * | 1-20 | A61L27/36<br>A61K35/39 |
| X,D | NAVARRO-TABLEROS VICTOR ET AL: "Islet-Like Structures Generated In Vitro from Adult Human Liver Stem Cells Revert Hyperglycemia in Diabetic SCID Mice", STEM CELL REVIEWS, HUMANA PRESS INC, US, vol. 15, no. 1, 6 September 2018 (2018-09-06), pages 93-111, XP036689720, ISSN: 1550-8943, DOI: 10.1007/S12015-018-9845-6 [retrieved on 2018-09-06] | 9-19 | A61P3/10 |
| Y | * page 95 *<br>* page 101 - page 102; figure 6 * | 1-20 | |
| Y | WO 2016/108237 A1 (ORGENESIS LTD [IL] ET AL.) 7 July 2016 (2016-07-07)<br>* claims 1-2 * | 1-20 | |
| Y | WO 2018/207179 A1 (ORGENESIS LTD [IL] ET AL.) 15 November 2018 (2018-11-15)<br>* page 1 - page 5 * | 1-20 | TECHNICAL FIELDS SEARCHED (IPC)<br><br>C12N<br>A61L<br>A61K<br>A61P |
| T | WO 2020/035480 A1 (UNICYTE ISLET AG [CH]) 20 February 2020 (2020-02-20) | | |

The present search report has been drawn up for all claims

| Place of search | Date of completion of the search | Examiner |
|---|---|---|
| Munich | 2 April 2020 | Paresce, Donata |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 19 20 6392

This annex lists the patent family members relating to the patent documents cited in the above-mentioned European search report.
The members are as contained in the European Patent Office EDP file on
The European Patent Office is in no way liable for these particulars which are merely given for the purpose of information.

02-04-2020

| Patent document cited in search report | | Publication date | Patent family member(s) | | Publication date |
|---|---|---|---|---|---|
| WO 2015091493 | A1 | 25-06-2015 | AU | 2014364632 A1 | 16-06-2016 |
| | | | CA | 2933135 A1 | 25-06-2015 |
| | | | CN | 105814193 A | 27-07-2016 |
| | | | EP | 3083941 A1 | 26-10-2016 |
| | | | JP | 6643993 B2 | 12-02-2020 |
| | | | JP | 2017500859 A | 12-01-2017 |
| | | | JP | 2020014477 A | 30-01-2020 |
| | | | KR | 20160098439 A | 18-08-2016 |
| | | | US | 2016312189 A1 | 27-10-2016 |
| | | | US | 2019119648 A1 | 25-04-2019 |
| | | | WO | 2015091493 A1 | 25-06-2015 |
| WO 2016108237 | A1 | 07-07-2016 | AU | 2015373129 A1 | 03-08-2017 |
| | | | BR | 112017014280 A2 | 27-03-2018 |
| | | | CA | 2972569 A1 | 07-07-2016 |
| | | | CN | 107750170 A | 02-03-2018 |
| | | | EA | 201791508 A1 | 31-05-2018 |
| | | | EP | 3240576 A1 | 08-11-2017 |
| | | | JP | 2018502574 A | 01-02-2018 |
| | | | KR | 20170102308 A | 08-09-2017 |
| | | | SG | 11201705344V A | 28-07-2017 |
| | | | US | 2016220616 A1 | 04-08-2016 |
| | | | US | 2019134097 A1 | 09-05-2019 |
| | | | WO | 2016108237 A1 | 07-07-2016 |
| WO 2018207179 | A1 | 15-11-2018 | EP | 3635106 A1 | 15-04-2020 |
| | | | WO | 2018207179 A1 | 15-11-2018 |
| WO 2020035480 | A1 | 20-02-2020 | NONE | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2015091493 A
- US 8178345 B
- US 7745209 B
- WO 2006126219 A
- WO 2006126236 A

### Non-patent literature cited in the description

- **REZANIA, A. et al.** Maturation of human embryonic stem cell-derived pancreatic progenitors into functional islets capable of treating pre-existing diabetes in mice. *Diabetes,* 2012, vol. 61 (8), 2016-2029
- **HERRERA, M. B. et al.** Isolation and characterization of a stem cell population from adult human liver. *Stem Cells.,* 2006, vol. 24, 2840-2850
- **TITUS K. et al.** Closed system cell culture protocol using HYPERStack vessels with gas permeable material technology. *J Vis Exp.,* 2010, vol. 45, 2499
- **BOSE B et al.** *Cell Biol Int.,* 2012, vol. 36 (11), 1013-20
- **RICORDI, C. et al.** Islet isolation assessment in man and large animals. *Acta Diabetologica Latina,* 1990, vol. 27, 185-195